# EUROPÄISCHE PATENTSCHRIFT

(11) **EP 0 437 774 B1**
(45) Veröffentlichungstag und Bekanntmachung des Hinweises auf die Patenterteilung: **05.03.1997**
(21) Anmeldenummer: 90124754.4
(22) Anmeldetag: 19.12.1990
(51) Int. Cl.: C07H 21/00, C12P 19/34, C12Q 1/68

(54) **Verfahren zur Herstellung von modifizierten Nukleinsäuren**
Process for the preparation of modified nucleic acids
Procédé pour la préparation d'acides nucléiques modifiés

(30) Priorität: 17.01.1990 DE 4001154
(43) Veröffentlichungstag der Anmeldung: 24.07.1991
(73) Patentinhaber: BOEHRINGER MANNHEIM GMBH, 68298 Mannheim (DE)
(72) Erfinder: Rüger, Rüdiger, Dr. med., W-8124 Seeshaupt (DE); Kessler, Christoph, Dr., W-8021 Dorfen (DE)

(56) Entgegenhaltungen:
- EP-A- 0 122 614
- EP-A- 0 139 489
- EP-A- 0 200 362
- EP-A- 0 201 184
- WO-A-86/07387
- WO-A-90/02205
- US-A- 4 851 331

## Beschreibung

Gegenstand der Erfindung ist ein Verfahren zur Herstellung von modifizierten Nukleinsäuren, ein Verfahren zum Nachweis von Nukleinsäuren, welches dieses Herstellungsverfahren umfaßt, Reagenzien zur Durchführung dieser Verfahren, sowie eine Nukleinsäure, welche in den genannten Verfahren hergestellt wird.

Verfahren zum Nachweis von Nukleinsäuren haben sich in der klinischen Diagnostik immer mehr zu einer empfindlichen Alternative zu Immuntests, beispielsweise auf dem Gebiet der Humandiagnostik, wie z. B. Virusdiagnostik und Bakteriendiagnostik, erwiesen. Jedoch auch in der Lebensmitteldiagnostik und der Histologie finden Verfahren zum Nachweis von Nukleinsäuren Verwendung. In diesen Verfahren wird das die nachzuweisende Nukleinsäure enthaltende Material mit einier zu der nachzuweisenden Nukleinsäure komplementären Nukleinsäure in Kontakt gebracht. Die Bildung eines Nukleinsäurehybrids kann anschließend auf verschiedene Weise sichtbar gemacht werden. Ein solches Verfahren ist beispielsweise in der DE-A-2801582 oder der US-A-4358535 beschrieben.

Diese Verfahren sind jedoch zum Nachweis sehr geringer Mengen an Nukleinsäure weniger gut geeignet. Zur Verbesserung dieser Verfahren wurde in der EP-A-0200362 vorgeschlagen, die nachzuweisende Nukleinsäure in einem der Hybridisierungsreaktion vorangehenden Schritt durch ein in-vitro-System zu vermehren. Dazu wird der Probe pro nachzuweisendem Nukleinsäure-Einzelstrang mindestens ein sogenannter Primer zugesetzt. Durch eine enzymatische Verlängerungsreaktion wird zu jedem der Nukleinsäure-Einzelstränge ausgehend von dem Primer durch Reaktion mit Mononukleotiden ein zu der template-Nukleinsäure komplementärer Nukleinsäurestrang gebildet. Diese Reaktion kann mehrmals hintereinander durchgeführt werden, wobei auch die neu gebildeten Nukleinsäurestränge vermehrt werden können. Wie die oben geschilderten Verfahren des Standes der Technik hat auch dieses Verfahren den Nachteil, daß im Anschluß an die Vermehrungsreaktion eine Hybridsierungsreaktion mit einer markierten Nukleinsäure ausgeführt wird.

In der EP-A-0 324 474 wird ein Blotting-Verfahren vorgeschlagen, bei dem wegen des Einbaus eines markierten Mononukleotids in die neu gebildeten Nukleinsäure auf eine Hybridisierung mit einer markierten Nukleinsäuresonde verzichtet werden kann. Dieses Verfahren hat jedoch den Nachteil, daß die Nukleinsäuren mittels Gelchromatographie aufgetrennt werden. Dies ist apparativ aufwendig und zeitintensiv.

In der Patentschrift US-4,851,331 wird ein Verfahren beschrieben, bei dem ein Primer an eine nachzuweisende Nukleinsäure hybridisiert und unter Einbau von markierten Mononukleotiden elongiert wird. Der Nachweis des gebildeten Nukleinsäurestranges erfolgt über radioaktiv markierte Mononukleotide, die in den Strang eingebaut wurden. Dieses Verfahren besitzt den Nachteil, daß eine Verwendung radioaktiver Materialien notwendig ist.

Aufgabe der vorliegenden Erfindung war es, die Nachteile des Standes der Technik zu vermeiden und insbesondere einfachere und weniger Schritte erfordernde besonders empfindliche Verfahren zum Nachweis von Nukleinsäuren bereitzustellen.

Gegenstand der Erfindung ist ein Verfahren zur Herstellung von Nukleinsäure durch Hybridisierung mindestens eines Primers an eine template-Nukleinsäure und enzymatische Verlängerung des Primers zu einem zu der template-Nukleinsäure komplementären Nukleinsäurestrang durch Reaktion mit verschiedenen Arten von Mononukleotiden, wobei mindestens eine Art von Mononukleotiden eine nachweisbare, biospezifische Gruppe und die gleiche oder mindestens eine andere Art von Mononukleotiden eine zur Immobilisierung befähigende biospezifische Gruppe trägt. Ebenfalls Gegenstand der Erfindung sind ein Verfahren zum Nachweis von Nukleinsäuren, ein Reagenz zur Herstellung von Nukleinsäuren, ein Reagenzkit zum Nachweis von Nukleinsäuren und eine in einem der obigen Verfahren hergestellte Nukleinsäure.

template-Nukleinsäuren im Sinne der Erfindung sind insbesondere DNA und RNA prokaryontischer oder eukaryontischer Herkunft. Dazu gehören auch virale und bakterielle Nukleinsäuren sowie Nukleinsäuren von Viroiden. Sie können einzel- oder doppelsträngig sein. Es kann sich um episomale Nukleinsäuren, wie Plasmide, oder genomische, chromosomale Nukleinsäuren handeln. Die Nukleinsäuren können für einen bestimmten Organismus oder eine Gruppe von Organismen charakteristisch sein. Es kann sich bei den Nukleinsäuren auch um schon vorbehandelte Nukleinsäuren handeln, beispielsweise um mittels Restriktionsenzymen geschnittene Nukleinsäuren. Im Falle von RNA sollte vorher cDNA hergestellt werden. Als vorteilhaft hat es sich erwiesen, wenn die Nukleinsäuren vor der Durchführung der Reaktion in einzelsträngiger Form vorliegen oder in einzelsträngige Form gebracht werden.

Als template-Nukleinsäuren werden im folgenden die in der Probe vorliegenden speziellen Nukleinsäuren, auf die sich die Nachweis- oder Herstellungsreaktion stützen soll, bezeichnet.

Die template-Nukleinsäure kann gereinigt vorliegen oder in einem Gemisch auch mit anderen Nukleinsäuren vorhanden sein, die nicht nachgewiesen oder benutzt werden sollen.

Diese Nukleinsäuren können in einer festen oder flüssigen, vorbehandelten oder nicht-vorbehandelten Probe enthalten sein. Bevorzugt sind die template-Nukleinsäuren in einer Probe enthalten, die durch Lyse eines Organismus, beispielsweise einer Zelle, erhalten wird und die eine Reihe verschiedenster Verbindungen, unter anderem auch andere Nukleinsäuren, enthält.

Zur Durchführung des erfindungsgemäßen Verfahrens zur Herstellung von Nukleinsäuren wird die Probe, welche eine template-Nukleinsäure enthält, unter geeigneten Bedingungen mit mindestens einem, bevorzugt einem Primer pro Nukleinsäureeinzelstrang versetzt. Dieser Primer ist bevorzugt ein Oligonukleotid von 12 bis 60, insbesondere 15 bis 30 nt (Nukleotiden) Länge. Der Primer kann mit einem bestimmten Bereich der template-Nukleinsäure hybridisieren. Dies ist möglich, wenn der Primer eine Nukleotidsequenz aufweist, welche im wesentlichen komplementär ist zu einem Bereich der template-Nukleinsäure.

Der Primer ist bevorzugt aus Deoxyribonukleotiden oder Ribonukleotiden aufgebaut, wobei diese Nukleotide modifiziert oder unmodifiziert sein können. Bevorzugt sind die meisten Nukleotide des Primers unmodifiziert.

Unmodifizierte Nukleotide sind die natürlich vorkommenden Nukleotide, beispielsweise Adenosin, Guanosin, Thymidin, Uridin und Cytidin. Modifizierte Nukleotide sind Nukleotidanaloge, wie 7-Desazaadenosin oder 7-Desazaguanosin, oder Derivate der genannten unmodifizierten Nukleotide, die eine zur Immobilisierung befähigende (immobilisierbare) oder eine nachweisbare Gruppe enthalten.

Zur Immobilisierung befähigende biospezifische Gruppen sind Gruppen bzw. Molekülteile, die über gruppenspezifische Wechselwirkungen von einem anderen Molekül oder Molekülteil erkannt und gebunden werden können. Solche Gruppen sind daher z.B. Haptene, Antigene und Antikörper, Glykoproteine, beispielsweise Lectine, oder auch die Bindungspartner von Bindeproteinen, wie Biotin oder Iminobiotin. Bevorzugt sind Haptene und Vitamine, besonders bevorzugt sind Biotin oder Steroide, wie Digoxigenin.

Nachweisbare biospezifische Gruppen sind beispielsweise kovalent gebundene, direkt nachweisbare Gruppen oder Molekülteile, wie Fluoreszenzfarbstoffe oder chromophore Substanzen, oder auch Gruppen, die über eine nachgeschaltete Reaktion indirekt nachweisbar sind. Dazu gehören insbesondere solche biospezifischen Gruppen, wie sie auch oben für die zur Immobilisierung befähigende Gruppen beschrieben sind. Besonders bevorzugt ist Digoxigenin.

Der Primer kann eine oder mehrere solche zur Immobilisierung befähigende oder nachweisbare Gruppen enthalten. Er kann auch sowohl eine oder mehrere zur Immobilisierung befähigende als auch eine oder mehrere nachweisbare Gruppen beinhalten. Bevorzugt enthält der Primer gar keine dieser Gruppen.

Solche Primer können auf dem Fachmann bekannte Weise hergestellt werden, beispielsweise durch chemische Synthese analog zu WO 84/03285 oder durch enzymatischen Einbau analog zu EP-A-0063879. Die Modifizierung kann an den Primer an beliebiger Stelle angebracht sein, bevorzugt jedoch nicht am 3'-Ende. Möglich ist, beispielsweise die Modifizierung am 5'-Ende (beispielsweise nach Molecular Cloning, S. 239 (1982) Ed. Maniatis et al., CSH) oder/und über die Nukleobasen, beispielsweise über einenε-Aminocapronsäurelinker und/oder über Random-priming nach Feinberg et al. (Anal. Biochem. 132, 6 (1983)). Prinzipiell sind auch alle Primer geeignet, die in der EP-A-0200362 beschrieben sind.

Die Bedingungen, unter welchen eine Hybridisierung des Primers mit dem dazu passenden Teil der template-Nukleinsäure stattfindet, sind dem Fachmann beispielsweise aus der EP-A-0200362 bekannt. Sie richten sich nach der Länge des Primers sowie dem Ausmaß der Komplementarität.

Der Primer sollte insbesondere an seinem 3'-Ende mindestens ein Nukleotid aufweisen, welches zu dem entsprechenden Nukleotid der template-Nukleinsäure komplementär ist. Der Primer kann, bevorzugt am 5'-Ende, außerdem eine Nukleotidsequenz enthalten, die nicht komplementär zu der template-Nukleinsäure ist. Er kann auch Erkennungsstellen für Enzyme, beispielsweise Polymerasen, enthalten; es können dazu auch Proteine gebunden sein.

Die Nukleotidsequenz des Primers wird so gewählt, daß die Einzelstrang-Nukleotidsequenz der template-Nukleinsäure nach der Hybridisierung über das 3'-Ende des Primers hinausgeht.

In dem erfindungsgemäßen Verfahren zur Herstellung von Nukleinsäuren wird die aus template-Nukleinsäure und Primer gebildete Hybrid-Nukleinsäure am 3'-Ende des Primers verlängert, indem ein zu dem oben genannten einzelsträngigen Bereich der template-Nukleinsäure komplementäres an den Primer anschließendes Nukleinsäurestück gebildet wird. Es kann hierbei analog zur EP-A-0200362 vorgegangen werden. Dort wird die Verlängerung mittels vierer Mononukleosidtriphosphate beschrieben.

Im erfindungsgemäßen Verfahren werden als Mononukleosidtriphosphate neben nicht modifizierten auch modifizierte Nukleosidtriphosphate eingesetzt. Modifizierte Nukleosidtriphosphate sind die Triphosphate der oben geschilderten modifizierten Nukleotide. Beispiele solcher modifizierter Mononukleosidtriphosphate sind Digoxigenin-dUTP (EP-A-0 324 474) oder Biotin-dUTP. Als modifizierte Nukleosidtriphosphate können in der Verlängerungsreaktion gleichzeitig solche Nukleosidtriphosphate, die eine zur Immobilisierung befähigende Gruppe tragen, und solche Nukleosidtriphosphate eingesetzt werden, die eine nachweisbare Gruppe tragen. Bei der zur Immobilisierung befähigenden Gruppe und der nachweisbaren Gruppe handelt es sich hier bevorzugt um unterschiedliche Gruppen. Bei einer Verlängerung um mehr als ca. 30 - 40 Nukleotide kann durch Einhaltung bestimmter Versuchsbedingungen gewährleistet werden, daß mehrere zur Immobilisierung befähigende Gruppen in die neu gebildete Nukleotidsequenz eingebaut werden. Bevorzugt sind nicht alle Nukleosidtriphosphate modifiziert. Besonders bevorzugt ist der Fall, daß neben den 4 natürlichen Nukleosidtriphosphatarten eine Nukleosidtriphosphatart in modifizierter Form eingesetzt wird.

Die unmodifizierte Menge des Nukleosidtriphosphats, das auch in modifizierter Form eingesetzt wird, wird bevorzugt um die Menge an eingesetztem modifziertem Nukleosidtriphosphat verringert, so daß die Gesamtmenge dieser Nukleosidtriphosphatart ungefähr erhalten bleibt. Die Menge der verschiedenen Mononukleosidtriphosphatarten ist jeweils ungefähr gleich und dem Fachmann bekannt. Sie beträgt beispielsweise bei Verlängerung analog zu EP-A-0 200 362 bevorzugt 100 µM - 300 µM, besonders bevorzugt ca. 200 µM.

Über folgende Ausführungsformen des erfindungsgemäßen Verfahrens ist es möglich, Nukleinsäuren herzustellen, welche mindestens eine nachweisbare biospezifische und mindestens eine zur Immobilisierung befähigende biospezifische Gruppe beinhalten und zu mindestens einem Teil einer template-Nukleinsäure komplementär sind:
- Einsatz eines Primers, der mindestens eine nachweisbare Gruppe enthält, und einer Nukleosidtriphosphatart, die eine immobilisierbare Gruppe enthält. Dabei kann der Primer zusätzlich noch eine oder mehrere immobilisierbare Gruppen enthalten und außerdem eine Nukleosidtriphosphatart eingesetzt werden, die eine nachweisbare Gruppe enthält. Bevorzugt sind jedoch die Ausführungsformen, bei denen der Primer nicht zusätzlich eine nachweisbare Gruppe aufweist.
- Einsatz eines Primers, der mindestens eine immobilisierbare Gruppe enthält, und Nukleosidtriphosphaten, die eine nachweisbare Gruppe enthalten, sowie Nukleosidtriphosphaten, die ein immobilisierbare Gruppe enhalten. Auch hier kann der Primer außerdem noch eine nachweisbare Gruppe enthalten.
- Einsatz eines Primers, welcher mehr als eine immobilisierbare Gruppe enthält, und Nukleosidtriphosphaten, die eine nachweisbare Gruppe enthalten. Zusätzlich können noch Nukleosidtriphosphate eingesetzt werden, die eine immobilisierbare Gruppe enthalten.
- Einsatz eines Primers, der keine immobilisierbare und keine nachweisbare Gruppe enthält, und Mononukleosidtriphosphaten, die eine nachweisbare Gruppe enthalten, sowie Mononukleosidtriphosphaten, die eine immobilisierbare Gruppe enthalten. Dieser Fall ist bevorzugt, da hier eine Reaktion zur Modifizierung des Primers wegfällt. Außerdem ist durch den Einbau sowohl nachweisbarer als auch immobilisierbarer Mononukleotide gewährleistet, daß die gebildete Nukleinsäure eine Vielzahl von immobilisierbaren Gruppen und mindestens eine nachweisbare Gruppe enthält. Ein weiterer Vorteil dieser Variante ist die erheblich erleichterte Abtrennbarkeit der gebildeten Nukleinsäuren von nicht umgesetzten Primern.

Als Enzym zur Verlängerung des Primers kommen Enzyme in Frage, die DNA- bzw. RNA-abhängige DNA- oder RNA-Polymerase-Aktivität aufweisen. Bevorzugt sind thermostabile DNA-Polymerasen. Dazu gehört beispielsweise Taq-DNA-Polymerase oder Klenow-Fragment der E. coli DNA-Polymerase.

Die Verlängerungsreaktion endet im allgemeinen bevorzugt am 5'-Ende der template-Nukleinsäure. Sie kann jedoch gewünschtenfalls auch schon vorzeitig durch Verwendung von Stopreagenzien, z.B. Stopnukleotiden, abgebrochen werden. Falls erforderlich wird zum Schluß das neugebildete Nukleinsäurestück an den Primer ligiert, beispielsweise über die Ligasereaktion.

Durch die enzymkatalysierte Reaktion wird der Primer unter Verwendung von modifizierten und unmodifizierten Mononukleosidtriphosphaten um ein Nukleinsäurestück verlängert, das komplementär zu dem entsprechenden Teil der template-Nukleinsäure ist. Die neu entstandene Nukleinsäure enthält dann zwei oder mehr zur Immobilisierung befähigende Gruppen und eine oder mehr nachweisbare Gruppen. Es hat sich herausgestellt, daß es sehr vorteilhaft ist, wenn zwei oder mehr zur Immobilisierung befähigende Gruppen in einem Strang der neu gebildeten Nukleinsäure enthalten sind. Insbesondere können solche Nukleinsäuren effizienter immobilisiert werden als solche, die nur eine solche Gruppe aufweisen. Dies erleichtert eine Quantifizierung der Ausgangsmenge an template-Nukleinsäure durch genauere Quantifizierung der neu gebildeten Nukleinsäuren.

Die Zahl der nachweisbaren Gruppen in jedem neugebildeten Strang ist bevorzugt größer als 1 und besonders bevorzugt weist ca. jedes 15. bis 30. Nukleotid eine nachweisbare Gruppe auf.

Mit dem erfindungsgemäßen Verfahren können beispielsweise Nukleinsäuren hergestellt werden, die eine Länge von 100 bis 8000 bp aufweisen. Sie ist jedoch praktisch nur begrenzt durch das Enzymsystem, welches die Verlängerung ausführt.

Je nach weiterer Anwendung der Amplifikationsprodukte kann die Polymerase beispielsweise durch Phenolisierung abgereinigt werden. Bei alleiniger Analytik im Southernblot, Dot Blot oder in der MTP ist dies nicht notwendig.

Bevorzugt werden die neugebildeten Nukleinsäuren als Doppel- oder als Einzelstrang von den nicht umgesetzten Mononukleosidtriphosphaten und Primern durch Gelchromatographie, an Affinitätsmaterialien oder durch Fällung abgetrennt. Dabei ist das erfindungsgemäße Verfahren, welches ohne mit einer immobilisierbaren Gruppe modifizierten Primer arbeitet, besonders vorteilhaft, da die Abtrennung der neu gebildeten Nukleinsäuren von unmodifizierten Primern an Festphasen, die die immobilisierbare Gruppe binden, besonders einfach ist.

Die gebildeten Nukleinsäuren können gewünschtenfalls von der template-Nukleinsäure getrennt werden, beispielsweise durch Denaturierung des Doppelstrangs oder über eine Replacement-Reaktion Die neu gebildeten Nukleinsäuren können Gegenstand weiterer Reaktionen sein, beispielsweise von Fragmentierungen durch Restriktionsenzyme, wie in der EP-A-0300796. Sie können auch selbst als template-Nukleinsäure zur Bildung von Nukleinsäuren in dem oben geschilderten erfindungsgemäßen Verfahren eingesetzt werden; diese Nukleinsäuren sind dann wiederum mindestens zum Teil zu ihnen komplementär und zu der ursprünglichen template-Nukleinsäure homolog. Als Primer muß dann anstelle einer zur template-Nukleinsäure komplementären Nukleotidsequenz ein mit der neu gebildeten Nukleinsäure hybridisierbarer Primer eingesetzt werden. Ansonsten muß er die oben geschilderten Bedingungen erfüllen. Da auch die ursprüngliche template-Nukleinsäure wieder als Matrize für die Bildung einer neuen Nukleinsäure zur Verfügung steht, ergibt sich eine zumindest annähernd exponentielle Vermehrung der beiden neu gebildeten Nukleinsäurestränge, wenn jede der Nukleinsäuren auf erfindungsgemäße Weise eingesetzt wird. Dies kann in einzelnen Temperatur- oder/und Reagenzzyklen, aber auch homogen geschehen. Prinzipiell kann die Reaktion zu jedem Zeitpunkt durch Zugabe von Stopreagenzien (beispielsweise EDTA) gestoppt werden. Außerdem ist es möglich, das erfindungsgemäße Verfahren dahingehend zu modifizieren, daß analog zu EP-A-0201184 nach einiger Zeit sogenannte "nested" Primer eingesetzt werden. Von diesem Zeitpunkt an wird dann nur noch ein Teil der bisher neu hergestellten Nukleinsäuresequenz amplifiziert.

Die Verfahren können auch so geführt werden, daß die zum Einbau zur Verfügung stehende Menge an zur Immobilisierung befähigendem Nukleosidtriphosphat in den letzten Reaktionszyklen limitiert wird. Dies hat den Vorteil, daß die nichteingebauten Bio-Nukleotide zum größten Teil in der Reaktion verbraucht sind, daher nicht mehr mit den biomarkierten Amplifikationsprodukten um SA-Bindungsstellen konkurrieren. Daher ist eine vorgeschaltete Abtrennung der nichteingebauten Nukleotide (Säule oder Fällung) nicht mehr notwendig.

Als template-Nukleinsäuren für das erfindungsgemäße Verfahren können auch Nukleinsäuren bzw. Fragmente davon eingesetzt werden, wie sie als Ergebnis von Nukleinsäure-Amplifikationsreaktionen, beispielsweise der EP-A-0310229, der EP-A-0300796, der WO 88/10315, der EP-A-0201184, der EP-A-0329822, der EP-A-0272098 oder der EP-A-0303155 entstehen. In diesen Fällen ist es möglich, sogar mit nur einmaliger Ausführung der Verlängerungsreaktion gemäß dem erfindungsgemäßen Verfahren eine große Menge an erfindungsgemäß modifizierten Nukleinsäuren herzustellen.

Eine besonders bevorzugte Ausführungsform des Verfahrens stellt die Durchführung des Verfahrens gemäß EP-A-0201184 unter Verwendung zweier unterschiedlich markierter Mononukleosidtriphosphate, beispielsweise Biotin-16-dUTP (immobilisierbar, Leary et al., Proc. Natl. Acad. Sci. USA 80, 4045-49 (1983)) und Digoxigenin-11-dUTP (nachweisbar), dar.

Als Versuchsbedingungen hierzu haben sich als besonders zweckmäßig erwiesen:

Volumen der Reaktion: 50-100 _{µ}l; Konzentration des markierten Primers 200 nM-1 _{µ}M; Gesamtkonzentration der dNTP-Arten: 100-300 _{µ}M; Taq-DNA-Polymerase: 1-3 U oder eine andere thermostabile Polymerase; Puffer: 30-100 mM KCl; 5-20 nM Tris, pH 8.2-8.7 bei RT; 0,5-2 mM MgCl₂; gegebenenfalls Stabilisatoren wie Gelatine oder Nuklease-freies BSA; PCR-Zyklen: 20-60, bevorzugt 20-30; Denaturierung: vor dem Start 2-10', 92-95°C (fakultativ; wenn hochkomplexe DNA), während PCR 1-5', 92-95°; Primer Hybridisierung 1-5', 35-55°C, Elongation: 1-10', 65-75°C, bevorzugt 3'. Trennung der nicht eingebauten dNTP's und Primer-Moleküle von den gebildeten Nukleinsäuren am Ende der PCR: Sephadex G 75 oder G 100 (Pharmacia) oder Streptavidin-Festphase (beispielsweise gemäß DE-A-3817716). Die modifizierten dNTP's können schon ab dem 1. Zyklus, aber auch erst in den letzten 3-5 Zyklen zugegeben werden. Letztere Vorgehensweise empfiehlt sich bei limitierender Zugabe von Bio-dUTP. Mengenverhältnis der für jeden Einzelstrang spezifischen Primer zueinander bevorzugt ca. 1:1. Verhältnis Dig-dUTP:Bio-dUTP:dTTP von 3:3:1 bis 1:1:3, bevorzugt 1:1:2 bis 1:1:1. Verhältnis Dig-dUTP (bzw. Bio-dUTP):dTTP (bei Verwendung eines modifizierten Primers): 3:1 bis 1:3 bevorzugt 1:2.

Die oben angegebenen Werte sind Richtwerte. Ein Fachmann kann ohne weiteres erkennen, ob im Einzelfall auch davon abweichende Bedingungen möglich sind.

Es war überraschend, daß es möglich war, auf erfindungsgemäße Weise Nukleinsäuren herstellen zu können, die besser immobiliserbar sind als erwartet und den Vorteil haben, auf einfache Weise quantitativ nachgewiesen werden zu können.

Diese Vorteile können besonders gut in Verfahren zum Nachweis von Nukleinsäuren angewendet werden. Weiterer Gegenstand der Erfindung ist daher ein Verfahren zum Nachweis von Nukleinsäuren durch Bildung eines zu mindestens einem Teil eines Stranges der nachzuweisenden Nukleinsäure komplementären Stranges, Bindung der gebildeten Nukleinsäure an eine feste Phase und Nachweis der gebildeten Nukleinsäure, wobei der komplementäre Strang durch Hybridisierung mindestens eines Primers an eine template-Nukleinsäure und enzymatische Verlängerung des Primers zu einem zu der template-Nukleinsäure komplementären Nukleinsäurestrang durch Reaktion mit verschiedenen Arten, bevorzugt mindestens vier Arten, von Mononukleotiden unter Verwendung der nachzuweisenden Nukleinsäure als template-Nukleinsäure gebildet wird und der komplementäre Nukleinsäurestrang mindestens eine zur Immobilisierung befähigende biospezifische Gruppe und mindestens eine nachweisbare biospezifische Gruppe trägt.

Als template-Nukleinsäure des erfindungsgemäßen Herstellungsverfahrens dient dabei die nachzuweisende Nukleinsäure bzw. ein Teil davon, die gegebenenfalls wie oben beschrieben, vorbehandelt, beispielsweise amplifiziert wurde.

Die als Ergebnis des Verfahrens gebildeten Nukleinsäurestränge können anschließend in sehr einfacher Weise nachgewiesen werden. Dazu können Doppelstränge gewünschtenfalls in Einzelstränge gespalten werden. Die gebildeten doppelt modifizierten Nukleinsäuren können beispielsweise gelchromatographisch nachgewiesen werden. Bevorzugt werden sie jedoch mit einer geeigneten Festphase in Kontakt gebracht und dort immobilisiert.

Die Art der Festphase richtet sich nach der zur Immobilisierung befähigenden Gruppe. Ist die immobilisierbare Gruppe beispielsweise ein Hapten, dann kann eine Festphase verwendet werden, die an ihrer Oberfläche Antikörper gegen dieses Hapten aufweist. Ist die immobilisierbare Gruppe ein Vitamin, wie z. B. Biotin, dann kann die Festphase bindende Proteine, wie Avidin oder Streptavidin immobilisiert enthalten. Die Immobilisierung über eine Gruppe an der modifizierten Nukleinsäure ist besonders vorteilhaft, da sie unter milderen Bedingungen stattfinden kann als beispielsweise Hybridisierungsreaktionen.

Bevorzugt wird zur Immobilisierung der gebildeten Nukleinsäuren die Reaktionsmischung nach Beendigung des Verfahrens zur Herstellung der Nukleinsäuren in ein Gefäß gefüllt, welches an seiner Oberfläche mit der immobilisierbaren Gruppe reagieren kann. Das Gefäß kann beispielsweise eine Küvette oder eine Miktrotiterplatte sein. Es ist jedoch auch möglich, eine Festphase in Form eines porösen Materials, wie einer Membran, eines Gewebes oder eines Vlieses, zu verwenden, auf welche die Reaktionsmischung aufgegeben wird. Ebenso ist die Verwendung von Perlen, sogenannten beads, möglich.

Nach einer Inkubationszeit, während der die Immobilisierungsreaktion stattfindet, wird die flüssige Phase aus dem Gefäß, dem porösen Material oder den pelletierten beads entfernt. Die Festphase kann anschließend mit einem geeigneten Puffer gewaschen werden, da die Bindung der erfindungsgemäßen Nukleinsäure an der Festphase sehr fest ist.

Die Menge der an die Festphase gebundenen modifizierten Nukleinsäure kann auf im Prinzip bekannte Weise bestimmt werden, wobei sich die durchzuführenden Schritte nach der Art der nachweisbaren Gruppe richten. Ist die nachweisbare Gruppe ein Hapten, so wird die modifizierte Nukleinsäure bevorzugt mit einem markierten Antikörper gegen das Hapten umgesetzt, wie analog in der EP-A-0 324 474 beschrieben. Die Markierung kann beispielsweise eine Enzymmarkierung, wie β-Galaktosidase, alkalische Phosphatase oder Peroxidase sein. Im Fall der Enzymmarkierung wird die Menge an Nukleinsäure über die meist photometrische, chemoluminometrische oder fluorometrische Verfolgung einer Reaktion des Enzyms mit einem chromogenen, chemoluminogenen oder fluorogenen Substrat gemessen.

Bei indirekt nachweisbaren Gruppen können die erforderlichen Reagenzien (beispielsweise ein gegen ein Hapten gerichteteter markierter Antikörper) auch schon vor dem Waschschritt zu der Reaktionsmischung zugegeben werden.

Der Nachweis der Nukleinsäure kann sowohl qualitativ als auch quantitativ geführt werden. Im Falle einer quantitativen Auswertung hat es sich als zweckmäßig herausgestellt, mindestens einen Vergleichsversuch mit einer Probe bekannten Nukleinsäuregehalts durchzuführen. Auch die Erstellung einer Eichkurve ist möglich und empfehlenswert.

Das erfindungsgemäße Nachweisverfahren kann auf allen in der EP-A-0 200 362 genannten Gebieten eingesetzt werden. Auch in der Virus- und Bakteriendiagnostik gemäß EP-A-0 229 701 und EP-A-0 131 052 ist das Verfahren einsetzbar.

Eine besonders bevorzugte Ausführungsform des erfindungsgemäßen Nachweisverfahrens umfaßt die besonders bevorzugte Ausführungsform des Herstellungsverfahrens und eine anschließende Detektion der neu gebildeten Nukleinsäure. Für die Detektion haben sich folgende Rahmenbedingungen als besonders vorteilhaft erwiesen:
Abtrennung der gebildeten Nukleinsäuren an einer Streptavidin-Oberfläche (DE-A-3817716) 1 bis 4 Stunden bei 37°C; Waschen mit Puffer; Inkubation mit Lösung eines AK gegen Digoxigenin, der mit alkalischer Phosphatase markiert ist; Waschen mit Puffer; Inkubation 4-Methylumbelliferyl-Phosphat, 0,05 bis 0,2 M; Messen in einem Fluoreszenzmeter; Vergleich gegen eine Eichkurve.

Ebenfalls Gegenstand der Erfindung ist eine einzel- oder doppelsträngige Nukleinsäure, dadurch gekennzeichnet, daß sie 1 oder mehrere nachweisbare biospezifische Gruppen und 2 oder mehr zur Immobilisierung befähigende biospezifische Gruppen in einem Nukleinsäurestrang aufweisen. Diese modifzierten Nukleinsäuren haben beispielsweise den Vorteil, daß sie sehr fest an geeigneten Festphasen immobilisierbar sind. Sie können unter Verwendung einer template-Nukleinsäure in erfindungsgemäßer Weise hergestellt werden. Sie können jedoch auch auf andere Weise hergestellt werden, beispielsweise durch Transkription analog zu DE-A-3726934, wenn entsprechende Mononukleosidtriphosphate (immobilisierbare und nachweisbare Gruppen) eingesetzt werden. Oft sind hierzu keine Primer erforderlich.

Weitere Gegenstände der Erfindung sind Reagenzkits zur Durchführung des erfindungsgemäßen Verfahrens zum Nachweis von Nukleinsäuren. Eines dieser Reagenzkits enthält in getrennten Behältern
- ein erstes Mononukleosidtriphosphat, das eine nachweisbare biospezifische Gruppe trägt
- ein zweites Mononukleosidtriphosphat, welches eine zur Immobilisierung befähigende biospezifische Gruppe trägt und
- ein Enzym, welches unter Verwendung einer template-Nukleinsäure und vier Mononukleotiden eine zu der template-Nukleinsäure komplementäre Nukleinsäure bilden kann.

Außerdem enthält es bevorzugt alle sonstigen Reagenzien, die zur Verlängerung von Primern zu Nukleinsäuren, die zu einem Nukleinsäurestrang komplementär sind, benötigt werden. Dazu gehören insbesondere mindestens ein Primer, welcher für die nachzuweisende Nukleinsäure oder Nukleinsäurespezies spezifisch ist sowie alle übrigen Mononukleosidtriphosphate. Außerdem kann es als Hilfsstoffe geeignet pH-Puffer-Substanzen, Denaturierungslösungen und Waschlösungen enthalten.

Es enthält außerdem bevorzugt eine spezifische Nukleotid-Sequenz, welche mit dem Primer hybridisieren kann, als Kontrolle.

Gegebenenfalls enthält das Reagenzkit auch die Reagenzien, welche zur Bestimmung der nachweisbaren Gruppe erforderlich sind. Im Falle der Verwendung von Haptenen ist beispielsweise bevorzugt ein markierter Antikörper in einem getrennten Behälter enthalten.

Ein anderes Reagenzkit enthält in getrennten Behältern:
- mindestens einen zu der nachzuweisenden Nukleinsäure im wesentlichen komplementären Primer, welcher mindestens eine zur Immobilisierung befähigende biospezifische Gruppe trägt,
- mindestens ein Mononukleosidtriphosphat, welches eine nachweisbare biospezifische Gruppe trägt und
- ein Enzym, welches unter Verwendung einer template-Nukleinsäure eines Primers und vier Mononukleotiden eine zur template-Nukleinsäure komplementäre Nukleinsäure bilden kann.

Auch es enthält bevorzugt alle sonstigen Reagenzien, die zur Verlängerung von Primern zu Nukleinsäuren benötigt werden. Dazu gehören hier insbesondere die übrigen Mononukleosidtriphosphate. Auch die genannten Hilfsstoffe, Kontrollnukleinsäuren und Reagenzien zum Nachweis der Gruppe sind bevorzugt darin enthalten.

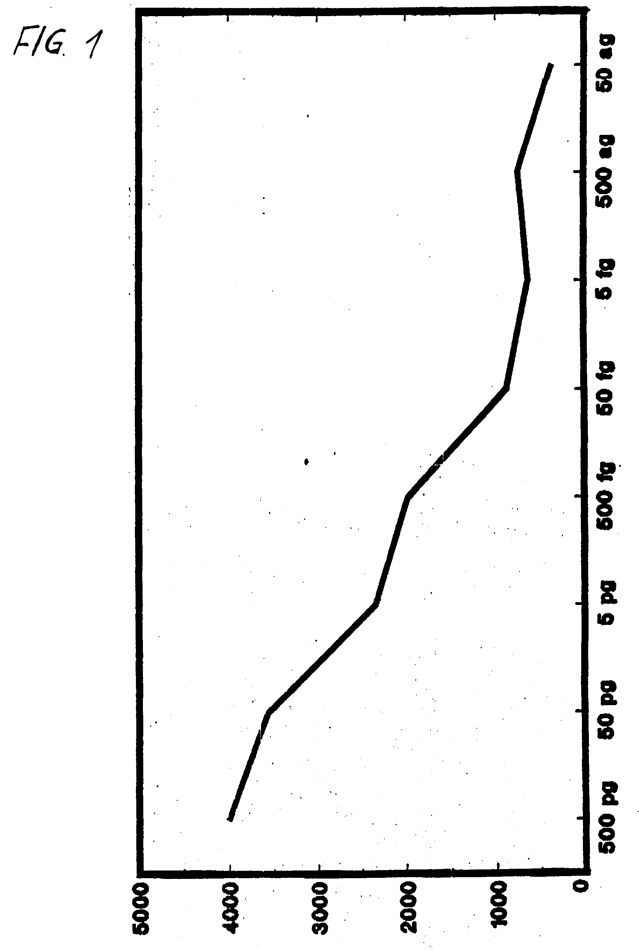

Fig. 1 zeigt das Ergebnis einer Bestimmung von Nukleinsäuren nach dem erfindungsgemäßen Verfahren. Diese Ergebnisse können auch als Eichkurve verwendet werden.

Die Erfindung wird durch die folgenden Beispiele weiter erläutert:

### Beispiel 1

### Polymerase-chain-reaction (PCR) mit zwei verschiedenen modifizierten Mononukleotidtriphosphaten

Verdünnungen von 10 ng - 100 ag des Plasmids pSPT18neo (template-Nukleinsäure, Herstellung gemäß Beck et al. in Gene 19:327-336 (1982)) werden für die PC-Reaktion gemäß EP-A-0200362 verwendet. pSPT18neo enthält die Sequenz des Neomycin(neo)-Gens in einem 940 bp SmaI-BglI-Fragment in der SmaI-BamHI-Site von pSPT 18. Das spezifisch amplifizierte Fragment in den Plasmid-Verdünnungen entspricht also 2,3 ng - 23 ag. Die Reaktion wird mit in diesem Plasmid die Neo-Sequenz flankierenden T7- und SP6-Promotor spezifischen Primern (Boehringer Mannheim, Best. Nr. 1175122 und 902152) gestartet. In einem Volumen von 50 _{µ}l werden 30 PCR-Zyklen in KCl, 50 mM; Tris HCl, pH 8,5, 10 mM; MgCl₂, 1,5 mM; Gelatine, 100 _{µ}g/ml; dATP, 200 _{µ}M; dCTP, 200 _{µ}M; dGTP, 200 _{µ}M; dTTP, 100 _{µ}M; Digoxigenin-11-2'-desoxyuridin-5'-dUTP (Dig-11-dUTP, EP-A-0324474), 50 _{µ}M; Biotin-16-2'-desoxyuridin-5'-triphosphat (Bio-16-dUTP, Boehringer Mannheim GmbH, Best.-Nr. 1093070), 50_{µ}M; mit T7-Promotor-spezifischem Primer, 300 nM; und SP6-Promotor-spezifischem Primer, 300 nM; mit den angegebenen pSPT18neo-Verdünnungen und 2,5 U Thermus aquaticus (Taq)-DNA-Polymerase durchgeführt.
- Zyklen:: Denaturierung: 2' 92°C
Primer-Hybridisierung: 2' 42°C
Elongation: 2'75°C

Die PCR wird in einem Thermocycler durchgeführt (DNA-Thermal-Cycler Perkin Elmer Cetus). Die Reaktionsvolumina sind zum Schutz vor Evaporation mit 30 _{µ}l Paraffin bedeckt.

Nach der PC-Reaktion werdendie PCR-Produkte mit 5 _{µ}l 4 M LiCl versetzt und in 250 _{µ}l Äthanol 30' bei -70°C gefällt, bei 15.000g 5'pelletiert, zweimal mit 70% Äthanol gewaschen, getrocknet, in 50 _{µ}l Tris, pH 7,5, 10 mM aufgenommen und in eine mit Streptavidin beschichtete Mikrotiterplatte pipettiert. Die Bindung der Biotin-Digoxigenin-markierten Moleküle an die Streptavidin-Festphase wird bei 37°C 2 Stunden durchgeführt. Nach der Wandbindungsreaktion wird mit 2x SSC 3x 10' bei 37°C und mit Konjugat-Puffer (Tris-HCl, pH 7,5, 100 mM; NaCl 0,9%; BSA, 1%; Pluronic T68, 0,5%) einmal 10'bei 37°C gewaschen. Anschließend wird mit 40 U <Digoxigenin>-Alkalische Phosphatase-Konjugat 30' bei 37°C inkubiert und darauf folgend 5x mit je 200 _{µ}l Tris HCl, 100 mM; und NaCl, 150 mM gewaschen. Zuletzt wird 30'bei 37°C mit 4-Methylumbelliferyl-Phosphat (0,1 mM) inkubiert und im Dynatech Microfluor-Reader gemessen.

In Figur 1 wird die Detektion von je 1/5 des Amplifikationsansatzes im Streptavidin-Tube gezeigt. Aufgetragen ist das FLuoreszenzsignal gegen die Konzentration an template-Nukleinsäure. Signale zwischen 100 und 500 fg sind ohne weiteres messbar.

### Beispiel 2

### PCR mit mehrfach modifizierten Primern

Die PCR wird mit dem gleichen Target in denselben Konzentrationen wie in Beispiel 1 durchgeführt. In der PC-Reaktion werden 30 Zyklen in KCl, 50 mM; Tris HCl, pH 8,5, 10 mM; MgCl₂, 1,5 mM; Gelatine 100 _{µ}g/ml; dATP, 200 _{µ}M, dCTP, 200 _{µ}M; dGTP, 200 _{µ}M; dTTP, 133 _{µ}M; Dig-11-dUTP, 66 _{µ}M; mit T7-Promotor-spezifischen Primern, 5'-(amidocaproyl)-Biotin-markiert mit N-(3-(N',N'-diisopropylaminomethoxyphosphinyloxy)hexyl)-2,2,2-trifluoroacetamid (Aminolink 2 von Applied Biosystems, USA) gemäß EP-A-0 261 283, 300 nM; und SP6-Promotor-spezifischem Primer, 5'-(amidocaproyl) Biotin-markiert via Aminolink 2, 300 nM; mit den in Beispiel 1 angegebenen pSPT18neo-Verdünnungen und 2,5 U Taq-DNA-Polymerase bei gleichen Zyklusprofilen durchgeführt. Nach PCR wird das Reaktionsvolumen mit Tris, pH 8,5, 10 mM; auf 300 _{µ}l aufgefüllt, in einer Sephadex G75-Säule bei 2.000 rpm 5' zentrifugiert und das Eluat mit 30 _{µ}l 4 M LiCl in 750 _{µ}l Äthanol 30'bei - 70°C gefällt, ebenso zentrifugiert und resuspendiert wie in Beispiel 1. Die Detektionsreaktion mit <Digoxigenin>-Alkalische Phosphatase-Konjugat wird wie in Beispiel 1 durchgeführt.

### Beispiel 3

### Bindung mehrfach Biotin-markierter Nukleinsäuren an Streptavidin

Sandwich-Hybridisierungs-Experimente werden mit rekombinanter Hepatitis-B-Virus (HBV) DNA als template (0, 1, 5, 10, 20, 40 und 80 ng), einem HBV-spezifischen Digoxigenin-markierten Oligonukleotid (40 Nukleotide, Digoxigenin-Markierung mit einem Digoxigenin-Molekül während Synthese) als Nachweisprobe und als Fangprobe entweder mit einem einfach Biotin-markiertem HBV-spezifischen Oligonukleotid (40 Nukleotide) oder einem sequenzgleichen mehrfach Biotin-markierten Oligonukletid (8 Biotinmoleküle/Oligonukleotid) durchgeführt. Die template-DNA wird vor der Hybridisierung 10 min. bei 100°C denaturiert und danach sofort auf Eis gebracht. Je 200 ng der Fang- bzw. Nachweisoligonukleotide werden in Natriumphosphat, pH 6,8, 50 mM; 2xSSC (NaCl, 300 mM; Na₃-Citrat 30 mM); 5x Denhardt'sche Lösung (0,1 % Polyvinylpyrrolidon; 0,1 % Bovines Serumalbumin; 0,1 % Ficoll 400) und mit den angegebenen Mengen an rekombinanter HBV-DNA in einem Gesamtvolumen von 200 _{µ}l im Streptavidin-beschichteten Tube 60 min bei 37°C hybridisiert.

Danach wird 2x 10 min bei 37°C mit 200 _{µ}l SSC, 2x/SDS, 0,2 % und 1x mit 0,9 % NaOH 10 min bei 37°C gewaschen. Anschließend folgt 30 min Inkubation mit 200 _{µ}l <Digoxigenin>-Meerrettich-Peroxidase-Konjugat (150 U/ml) bei 37°C in Tris-HCl, pH 7,5, 100 mM; NaCl 0,9 %; BSA, 1 %; Pluronic T68 0,5 % und 5x Waschen mit 0,9 % NaCl. Anschließend wird mit 0,1 % 2,2-Azino-di-{3-ethylbenzthiazolin-sulfonat (6)] (ABTS, BM Nr. 756407) in 200 _{µ}l ABTS-Puffer (BM-Nr. 1112597) inkubiert und die Extinktion bei 405 nm gemessen.

In Tabelle I ist zu sehen, daß die meßbare Extinktion (d.h. die Zahl der vorhandenen Streptavidin-gebundenen Sandwichmoleküle) bei Verwendung von mehrfach-markierten Fangprobes höher ist (bei sonst völlig identischen Bedingungen). Das weist auf eine effizientere Bindung der mehrfach Biotin-markierten Moleküle hin.

**Tabelle 1**

| HBV-Template ng | Extinktion Oligo-Fangprobe 1 Biotinmolekül | Extinktion Oligo-Fangprobe 8 Biotinmoleküle |
|---|---|---|
| 0 | 121 | 113 |
| 1 | 126 | 135 |
| 5 | 135 | 148 |
| 10 | 146 | 180 |
| 20 | 179 | 232 |
| 40 | 231 | 332 |
| 80 | 319 | 672 |

## Patentansprüche

1. Verfahren zur Herstellung von Nukleinsäuren durch Hybridisierung mindestens eines Primers an eine template-Nukleinsäure und enzymatische Verlängerung des Primers zu einem zu der template-Nukleinsäure komplementären Nukleinsäurestrang durch Reaktion mit verschiedenen Arten von Mononukleotiden, dadurch gekennzeichnet, daß mindestens eine Art von Mononukleotiden eine nachweisbare, biospezifische Gruppe und die gleiche oder mindestens eine andere Art von Mononukleotiden eine zur Immobilisierung befähigende, biospezifische Gruppe trägt.

2. Verfahren gemäß Anspruch 1, dadurch gekennzeichnet, daß der Primer ein oder mehrere zur Immobilisierung befähigende biospezifische Gruppen trägt.

3. Verfahren gemäß einem der Ansprüche 1 oder 2, dadurch gekennzeichnet, daß sowohl die hergestellte Nukleinsäure als auch die ursprüngliche template-Nukleinsäure als template-Nukleinsäuren zur Bildung weiterer Nukleinsäuren eingesetzt werden.

4. Verfahren zum Nachweis von Nukleinsäuren durch Bildung eines zu mindestens einem Teil eines Stranges der nachzuweisenden Nukleinsäure komplementären Stranges, Bindung der gebildeten Nukleinsäure an eine feste Phase und Nachweis der gebildeten Nukleinsäure, dadurch gekennzeichnet, daß der komplementäre Strang durch Hybridisierung mindestens eines Primers an eine template-Nukleinsäure und enzymatische Verlängerung des Primers zu einem zu der template-Nukleinsäure im wesentlichen komplementären Nukleinsäurestrang durch Reaktion mit verschiedenen Arten von Mononukleotiden unter Verwendung der nachzuweisenden Nukleinsäure als template-Nukleinsäure gebildet wird, dadurch gekennzeichnet, daß mindestens eine Art von Mononukleotiden eine nachweisbare, biospezifische Gruppe und die gleiche oder mindestens eine andere Art von Mononukleotiden eine zur Immobilisierung befähigende, biospezifische Gruppe trägt.

5. Reagenz zur Herstellung von Nukleinsäuren, enthaltend ein Mononukleosidtriphosphat, welches eine nachweisbare, biospezifische Gruppe trägt, und ein Mononukleosidtriphosphat, welches eine zur Immobilisierung befähigende biospezifische Gruppe trägt.

6. Reagenzkit zum Nachweis von Nukleinsäuren, enthaltend in getrennten Behältern:
- ein erstes Mononukleosidtriphosphat, das eine nachweisbare, biospezifische Gruppe trägt
- ein zweites Mononukleosidtriphosphat, welches eine zu Immobilisierung befähigende, biospezifische Gruppe trägt und
- ein Enzym, welches unter Verwendung einer template-Nukleinsäure und vier Mononukleotiden eine zu der template-Nukleinsäure komplementäre Nukleinsäure bilden kann.

7. Reagenzkit gemäß Anspruch 6, dadurch gekennzeichnet, daß es außerdem eine Kontrollnukleinsäure enthält.

8. Verfahren zur Herstellung modifizierter Nukleinsäuren durch Hybridisierung mindestens eines Primers an eine template-Nukleinsäure und enzymatische Verlängerung des Primers zu einem zu der template-Nukleinsäure komplementären Nukleinsäurestrang durch Reaktion mit verschiedenen Arten von Mononukleotiden, dadurch gekennzeichnet, daß als biospezifisch nachweisbar modifiziertes Mononukleosidtriphosphat Dig-dUTP als biospezifisch immobilisierbar modifiziertes Mononukleosidtriphosphat Bio-dUTP und als unmodifiziertes Mononukleosidtriphosphat dTTP im Verhältnis 3:3:1 bis 1:1:3, bevorzugt 1:1:2 bis 1:1:1 eingesetzt wird.

9. Verfahren gemäß Anspruch 1, bei dem mindestens eine Nukleotidtriphosphatart mit Biotin als biospezifisch immobilisierbare Gruppe eingesetzt wird.

10. Verfahren gemäß Anspruch 1, bei dem mindestens eine Nukleosidtriphosphatart mit Digoxigenin als biospezifisch nachweisbare Gruppe eingesetzt wird.

11. Verfahren gemäß Anspruch 1, bei dem der Primer keine nachweisbare oder zur Immobilisierung befähigende Gruppen beinhaltet.

## Claims

1. Process for the production of nucleic acids by the hybridization of at least one primer to a template nucleic acid and enzymatic elongation of the primer to a nucleic acid strand which is complementary to the template nucleic acid by reaction with different types of mononucleotides, wherein at least one type of mononucleotide carries a detectable biospecific group and the same or at least one other type of mononucleotide carries a biospecific group which enables the immobilization.

2. Process as claimed in claim 1, wherein the primer carries one or several biospecific groups which enable the immobilization.

3. Process as claimed in one of the claims 1 or 2, wherein the nucleic acid produced as well as the original template nucleic acid are used as template nucleic acids for the formation of further nucleic acids.

4. Process for the detection of nucleic acids by formation of a strand which is complementary to at least part of the strand of the nucleic acid to be detected, binding of the nucleic acid formed to a solid phase and detection of the nucleic acid formed, wherein the complementary strand is formed by hybridization of at least one primer to a template nucleic acid and enzymatic elongation of the primer to a nucleic acid strand which is essentially complementary to the template nucleic acid by reaction with different types of mononucleotides using the nucleic acid to be detected as the template nucleic acid, wherein at least one type of mononucleotide carries a detectable biospecific group and the same or at least one other type of mononucleotide carries a biospecific group which enables the immobilization.

5. Reagent for the production of nucleic acids containing a mononucleoside triphosphate which carries a detectable biospecific group and a mononucleoside triphosphate which carries a biospecific group which enables the immobilization.

6. Reagent kit for the detection of nucleic acids containing in separated containers:
- a first mononucleoside triphospate which carries a detectable biospecific group
- a second mononucleoside triphosphate which carries a biospecific group which enables the immobilization and
- an enzyme which, by use of a template nucleic acid and four mononucleotides, can form a nucleic acid which is complementary to the template nucleic acid.

7. Reagent kit as claimed in claims 6, wherein it in addition contains a control nucleic acid.

8. Process for the production of modified nucleic acids by hybridization of at least one primer to a template nucleic acid and enzymatic elongation of the primer to a nucleic acid strand which is complementary to the template nucleic acid by reaction with different types of mononucleotides, wherein Dig-dUTP is used as the modified biospecifically detectable mononucleoside triphosphate, Bio-dUTP is used as the modified biospecifically immobilizable mononucleoside triphosphate and dTTP is used as the unmodified mononucleoside triphosphate at a ratio of 3:3:1 to 1:1:3 preferably 1:1:2 to 1:1:1.

9. Process as claimed in claim 1, in which at least one type of nucleotide triphosphate is used with biotin as the biospecifically immobilizable group.

10. Process as claimed in claim 1, in which at least one type of nucleotide triphosphate is used with digoxigenin as the biospecifically detectable group.

11. Process as claimed in claim 1, in which the primer contains no detectable group or a group that enables the immobilization.

## Revendications

1. Procédé de préparation d'acides nucléiques par hybridation d'au moins une amorce à un acide nucléique matrice et allongement enzymatique de l'amorce en un brin d'acide nucléique complémentaire d'un acide nucléique matrice par réaction avec différents types de mononucléotides, caractérisé par le fait qu'au moins un type de mononucléotide porte un groupe détectable, biospécifique et le même type de mononucléotide, ou au moins un autre type de mononucléotide, porte un groupe biospécifique permettant l'immobilisation.

2. Procédé selon la revendication 1, caractérisé par le fait que l'amorce porte un ou plusieurs groupes biospécifiques permettant l'immobilisation.

3. Procédé selon l'une des revendications 1 ou 2, caractérisé par le fait que l'acide nucléique préparé, aussi bien que l'acide nucléique matrice initial, sont mis en oeuvre en tant qu'acide nucléique matrice pour la formation d'autres acides nucléiques.

4. Procédé de détection d'acides nucléiques par formation d'un brin complémentaire d'au moins une partie d'un brin de l'acide nucléique à détecter, fixation de l'acide nucléique formé sur une phase solide et détection de l'acide nucléique formé, caractérisé par le fait que le brin complémentaire est formé par hybridation d'au moins une amorce à un acide nucléique matrice et allongement enzymatique de l'amorce en un acide nucléique essentiellement complémentaire de l'acide nucléique matrice, par réaction avec différents types de mononucléotides, en utilisant l'acide nucléique à détecter en tant qu'acide nucléique matrice, caractérisé par le fait qu'au moins un type de mononucléotide porte un groupe biospécifique détectable et le même type de mononucléotide, ou au moins un autre type de mononucléotide, porte un groupe biospécifique, permettant l'immobilisation.

5. Réactif destiné à la préparation d'acides nucléiques, contenant un mononucléosidetriphosphate qui porte un groupe biospécifique détectable, et un mononucléosidetriphosphate qui porte un groupe biospécifique permettant l'immobilisation.

6. Trousse de réactifs destinée à la détection d'acides nucléiques, contenant, dans des récipients séparés :
- un premier mononucléosidetriphosphate qui porte un groupe biospécifique détectable,
- un deuxième mononucléosidetriphosphate qui porte un groupe biospécifique permettant l'immobilisation, et
- une enzyme qui peut former, en utilisant un acide nucléique matrice et quatre mononucléotides, un acide nucléique complémentaire de l'acide nucléique matrice.

7. Trousse de réactifs selon la revendication 6, caractérisée par le fait qu'elle contient en outre un acide nucléique témoin.

8. Procédé de préparation d'acides nucléiques modifiés, par hybridation d'au moins une amorce à un acide nucléique matrice et allongement enzymatique de l'amorce en un brin d'acide nucléique complémentaire de l'acide nucléique matrice, par réaction avec différents types de mononucléotides, caractérisé par le fait qu'en tant que mononucléosidetriphosphate modifié, détectable, biospécifique, on utilise du Dig-dUTP, en tant que mononucléosidetriphosphate modifié, immobilisable, biospécifique, on utilise du Bio-dUTP et en tant que mononucléosidetriphosphate non modifié, on utilise du dTTP, dans un rapport de 3 : 3 : 1 à 1 : 1 : 3, de préférence de 1 : 1 : 2 à 1 : 1 : 1.

9. Procédé selon la revendication 1, dans lequel au moins un type de nucléotidetriphosphate est utilisé avec de la biotine en tant que groupe biospécifique immobilisable.

10. Procédé selon la revendication 1, dans lequel au moins un type de nucléotidetriphosphate est utilisé avec de la digoxigénine en tant que groupe biospécifique immobilisable.

11. Procédé selon la revendication 1, dans lequel l'amorce ne comporte pas de groupe détectable ou permettant l'immobilisation.
